(19)

Europäisches
Patentamt
European
Patent Office
Office européen
des brevets

(11) **EP 4 565 301 B1**

(12) **EUROPEAN PATENT SPECIFICATION**

(45) Date of publication and mention
of the grant of the patent:
**10.06.2026 Bulletin 2026/24**

(21) Application number: **23761294.0**

(22) Date of filing: **02.08.2023**

(51) International Patent Classification (IPC):
***A61M 13/00*** *(2006.01)*

(52) Cooperative Patent Classification (CPC):
**A61M 13/003;** A61M 16/0063; A61M 16/202;
A61M 2205/3334; A61M 2205/3344; A61M 2205/50

(86) International application number:
**PCT/US2023/029270**

(87) International publication number:
**WO 2024/030469 (08.02.2024 Gazette 2024/06)**

(54) **MULTIMODAL SURGICAL GAS DELIVERY SYSTEM HAVING CONTINOUS PRESSURE MONITORING**

MULTIMODALES CHIRURGISCHES GASABGABESYSTEM MIT KONTINUIERLICHER DRUCKÜBERWACHUNG

SYSTÈME DE DISTRIBUTION MULTIMODALE DE GAZ CHIRURGICAL COMPORTANT UNE SURVEILLANCE CONTINUE DE LA PRESSION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **05.08.2022 US 202263395597 P**

(43) Date of publication of application:
**11.06.2025 Bulletin 2025/24**

(73) Proprietor: **CONMED Corporation**
**Largo, FL 33773 (US)**

(72) Inventors:
• **KOLTZ, Michael, L.**
**Aurora, CO 80016 (US)**
• **SELL, Leo**
**Centennial, CO 80111 (US)**

(74) Representative: **Hoefer & Partner Patentanwälte mbB**
**Pilgersheimer Straße 20**
**81543 München (DE)**

(56) References cited:
**US-A1- 2013 267 779     US-A1- 2018 221 598**
**US-A1- 2020 147 292     US-B1- 6 261 227**

**Description**

## CROSS-REFERENCE TO RELATED APPLICATIONS

The subject application claims the benefit of priority to U.S. Provisional Patent Application No. 63/395,597 filed August 5, 2022.

## BACKGROUND OF THE INVENTION

### 1. Field of the Invention

The subject invention is directed to endoscopic surgery, and more particularly, to a surgical gas delivery system that is adapted and configured to continuously monitor abdominal pressure during a surgical procedure without intentionally interrupting gas flow to or from the abdominal cavity.

### 2. Description of Related Art

Laparoscopic or "minimally invasive" surgical techniques are becoming commonplace in the performance of procedures such as cholecystectomies, appendectomies, hernia repair and nephrectomies. Such procedures commonly involve filling or "insufflating" the abdominal cavity with a pressurized fluid, such as carbon dioxide, to create an operating space, which is referred to as a pneumoperitoneum.

Insufflators are used to deliver pressurized insufflation gas to a patient's abdominal cavity during a surgical procedure, including insufflators those that are adapted and configured to recirculate insufflation gas through a recirculation circuit as disclosed in commonly assigned U.S. Patent No. 10,639,434. In this device, abdominal pressure is measured periodically through an insufflation path that communicates with the abdominal cavity by way of a trocar. The gas delivery device adjusts gas flow based on these periodic pressure measurements. It would be more beneficial however to monitor abdominal pressure continuously so that gas flow can be continuously adjusted throughout the system to maintain a more stable pneumoperitoneum.

Commonly assigned U.S. Patent No. 11,033,299 to Silver discloses a method of continuously monitoring body cavity pressure using a look up table that incudes data for continuous flow of insufflation gas delivered at variable rates and specified pressure, or at specified flow rates and variable driving pressure to infer body cavity pressure

U.S. Patent Application 2021/0346592 to Zeyssig et al. discloses a method for determining and controlling internal body pressure that includes controlling the output of a fluid pump by way of an estimated pressure value derived from instrument resistance coefficients and their associated curves stored in a memory device of the pump.

Further, US 2018/221598 A1 discloses a multimodal surgical gas delivery system according to the preamble part of claim 1.

The device and method described within this disclosure provide a way to continuously infer an insufflated body cavity pressure in a multimodal gas delivery system using a polynomial function consisting of as few as three terms and input from as few as three sensors. This method eliminates the need for storing characteristic curve data and lookup tables, which both consume more system memory and are more prone to error than the method described within this disclosure.

## SUMMARY OF THE DISCLOSURE

The subject disclosure is directed to a new and useful non-claimed method of monitoring insufflated body cavity pressure, which includes the step of repeatedly calculating body cavity pressure using a polynomial function of at least three relatively weighted terms including an insufflation gas supply pressure that is greater than the body cavity pressure, an insufflation gas flow rate and a compressor discharge pressure, and then adjusting insufflation gas flow and compressor discharge flow to maintain the body cavity pressure within a desired range. The method further includes the step of repeatedly measuring the insufflation gas supply pressure, the insufflation gas flow rate and the compressor discharge pressure.

The polynomial function includes first order terms, while in other embodiments of the subject disclosure the polynomial function includes second order or higher order terms. It is envisioned that the polynomial function could also include interaction terms to reduce predictive error.

The subject disclosure of the invention is directed to a multimodal surgical gas delivery system for monitoring insufflated body cavity pressure, which includes an insufflation portion defining an insufflation gas flow path for receiving insufflation gas from a gas supply by way of a pressure regulator and for delivering the insufflation gas to a patient's body cavity by way of a gas sealed surgical access device, and a gaseous sealing portion defining a gas recirculation circuit for communicating with the surgical access device to generate a gaseous seal therein for maintaining a stable body cavity pressure.

The insufflation gas flow path includes a gas control valve downstream from the pressure regulator, and it further includes an insufflation gas flow sensor and an insufflation gas pressure sensor downstream from the gas control valve. The gas recirculation circuit includes a compressor having a discharge path and a return path, wherein a discharge pressure sensor is operatively associated with the compressor discharge path.

The gas delivery system further includes a processor operatively associated with the insufflation portion and the gaseous sealing portion for repeatedly calculating body cavity pressure by way of the surgical access device using a polynomial function of at least three relatively weighted terms including an insufflation gas supply pressure measured by the insufflation gas pressure sensor, an insufflation gas flow rate measured by the insufflation gas flow sensor and a compressor discharge pressure measured by the discharge pressure sensor. The processor is adapted and configured to adjust gas flow through the gas control valve and the compressor to maintain the body cavity pressure within a desired range. For example, within a range of between 0.1 and 20 mmHg.

In one embodiment, the insufflation gas pressure sensor is located upstream from the insufflation gas flow sensor, while in another embodiment the insufflation gas pressure sensor is located downstream from the insufflation gas flow sensor. Alternatively, the insufflation portion could have two gas pressure sensors including a first gas pressure sensor located upstream from the gas flow sensor and a second gas pressure sensor located downstream from the gas flow sensor.

These and other features of the system and method of the subject disclosure will become more readily apparent from the following detailed description of the disclosed embodiments taken in conjunction with the drawings. The invention is defined by the appended claims.

## BRIEF DESCRIPTION OF THE DRAWINGS

So that those skilled in the art will readily understand how to make and use the gas delivery system of the subject disclosure without undue experimentation, preferred embodiments thereof will be described in detail herein below with reference to the figures wherein:

Fig. 1 is a schematic representation of the multimodal gas delivery system of the subject disclosure, which includes an insufflation path and a gas recirculation circuit; and

Fig. 2 is a plot of patient pressure (mmHg) measured in the insufflation path versus jet pressure (psi) measured in the gas recirculation circuit to illustrate the pressure contribution of the gas jets used to generate a gaseous seal within the surgical access device associated with the gas delivery system of Fig. 1.

## DETAILED DESCRIPTION OF THE EMBODIMENTS

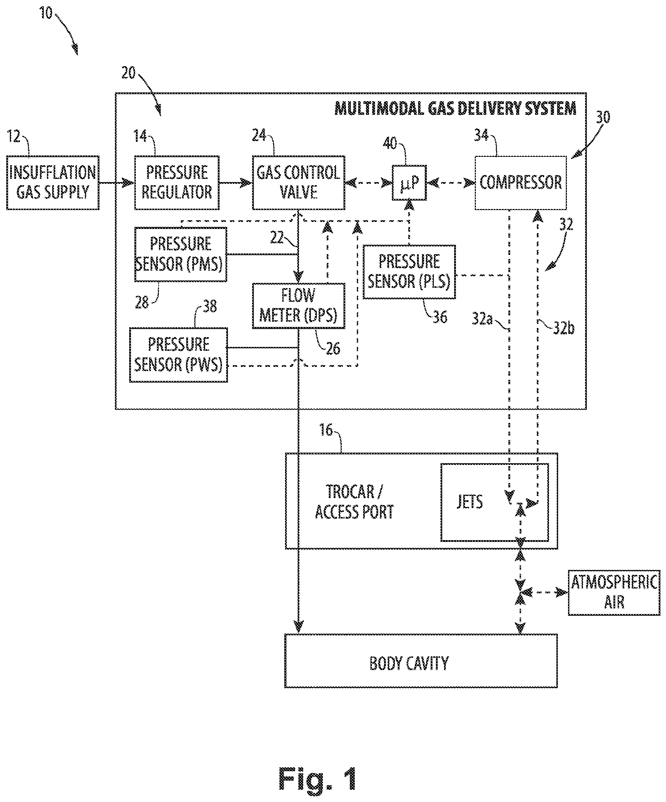

Referring now to the drawings wherein like reference numerals identify similar features or aspects of the subject disclosure, there is illustrated in Fig. 1 a new and useful multimodal surgical gas delivery system designated generally by reference numeral 10, which is adapted and configured to continuously monitor insufflated body cavity pressure. In other words, the insufflated body cavity pressure is monitored without intentionally interrupting gas flow to or from the abdominal cavity, including stopping the flow of gas to take periodic abdominal pressure measurements. An example of a multimodal gas delivery system of the type in which continuous insufflated body cavity pressure could be monitored is disclosed in commonly assigned U.S. Patent No. 9,199,047.

Referring now to Fig. 1, the surgical gas delivery system 10 includes an insufflation portion or manifold 20 defining an insufflation gas flow path or circuit 22 for receiving insufflation gas from a gas supply 12 by way of a pressure regulator 14 and for delivering the insufflation gas to a patient's body cavity by way of a gas sealed surgical access device 16. The gas supply 12 can be a portable gas bottle or tank or the gas could be supplied form a remote source of house gas. An example of a gas sealed surgical access device is disclosed in commonly assigned U.S. Patent No. 8,795,223.

The insufflation gas flow path 22 of insufflation portion 20 includes a gas control valve 24 located downstream from the pressure regulator 14. Gas flow path 22 further includes an insufflation gas flow sensor or meter (DPS) 26 and an insufflation gas pressure sensor (PLS) 28 downstream from the gas control valve 24.

The gas delivery system 10 further includes a gaseous sealing portion 30 defining a gas recirculation circuit 32 for communicating with the surgical access device 16 to generate a gaseous seal therein for maintaining a stable body cavity pressure. The gas recirculation circuit 32 includes a compressor or pump 34 having a discharge path 32a and a return path 32b. The compressor 34 provides gas pressure and flow to a jet assembly located within the access port 16 to develop a gaseous seal within the access port 16. This serves to maintain a stable pressure with the patient's body cavity. A discharge pressure sensor (PLS) 36 is operatively associated with the compressor discharge path 34b of recirculation circuit 32.

A controller or processor 40 is operatively associated with the insufflation portion 20 and the gaseous sealing portion 30 for repeatedly or otherwise periodically calculating body cavity pressure by way of the surgical access device 16.

EP 4 565 301 B1

In accordance with the subject disclosure, this is accomplished using a polynomial function of at least three relatively weighted terms including an insufflation gas supply pressure repeatedly or otherwise periodically measured by the insufflation gas pressure sensor 28, an insufflation gas flow rate repeatedly or otherwise periodically measured by the insufflation gas flow sensor 26 and a compressor discharge pressure repeatedly or otherwise periodically measured by the discharge pressure sensor 36.

Based on these calculations, the processor 40 is adapted and configured to adjust gas flow through the gas control valve 24 and the compressor 34 in real time to maintain the body cavity pressure within a desired range. This serves to maintain a stable pneumoperitoneum throughout the surgical procedure which is beneficial to the patient and desirable for the surgeon.

In one embodiment, the insufflation gas pressure sensor 28 is located upstream from the insufflation gas flow sensor 26, while in another embodiment the insufflation gas pressure sensor (PWS) 38 is located downstream from the insufflation gas flow sensor 26. Alternatively, the insufflation portion 20 of the gas delivery device 10 could have two gas pressure sensors including a first gas pressure sensor 28 located upstream from the gas flow sensor 26 and a second gas pressure sensor 38 located downstream from the gas flow sensor.

Gas is supplied to the multimodal gas delivery system 10 from the gas source 12 at a pressure that is greater than the insufflated body cavity pressure. The body cavity pressure is developed by the external compressed gas source 12 and the pneumo-dynamic effects of the jets within the access port 16, as described in commonly assigned U.S. Patent No. 9,907,569. Cavity pressure increases with both an increase in jet supply pressure as well as flow rate from the external compressed gas source 12.

As noted above, the gas delivery system 10 includes a controller or processor 40 that repeatedly or otherwise periodically calculates the body cavity pressure based on the pressure transducer and flow meter signals. The computer-implemented body cavity pressure calculations are preferably done periodically at a defined polling frequency in the range of between 1 GHz and 0.01 Hz. For example, the polling frequency for body cavity pressure calculations can be 1 kHz. This is advantageously done without intentionally interrupting gas flow to or from the abdominal cavity, including stopping the flow of gas to take periodic abdominal pressure measurements.

The calculation uses a polynomial of at least three terms as shown below in Equation 1.0 with the intercept concept being optional.

$$P_{cavity} = C_0 + C_{PMS} \cdot P_{PMS} + C_{DPS} \cdot Q_{DPS} + C_{PLS} \cdot P_{PLS}$$

Where:

$P_{cavity}$ is the Body Cavity Pressure;

$C_0$ is the Intercept Constant;

$C_{PMS}$ is the Constant for the PMS Pressure Sensor (28);

$P_{PMS}$ is the Pressure measured by the PMS Pressure Sensor (28);

$C_{DPS}$ is the Constant for the DPS Flow Meter (26);

$Q_{DPS}$ is the Flow measured by the DPS Sensor (26);

$C_{PLS}$ is the Constant for the PLS Pressure sensor (36); and

$P_{PLS}$ is the Pressure measured by the PLS Sensor (36).

Equation 1.0

The constants of the polynomial are derived from empirical data across expected variation of use conditions, i.e. pressures and flow rates, as well as part-to-part manufacturing variation for instruments and components. Multi-variate regression is used to derive the terms and constants of the polynomial. Terms are chosen based on their individual statistical significance in influencing the response, i.e. body cavity pressure.

The polynomial form of Equation 1.0 is one embodiment of the subject disclosure. However, other embodiments incorporating square or higher order terms as well as interaction terms can be used to reduce the predictive error of the body cavity pressure calculation.

Table 1.0

| $C_0$ | -0.5791367 |
|---|---|
| $C_{PMS}$ | 0.5949516 |
| $C_{DPS}$ | 0.0002156 |
| $C_{PLS}$ | 0.1736789 |

Examples of multi-variate regression derived coefficients are shown above in Table 1.0. Those skilled in the art will readily appreciate that coefficient values can change significantly in both sign and magnitude with different data sets obtained from identical system designs due to manufacturing and environmental variation. While coefficients can vary across a wide range, the body cavity pressure range is generally between 0.1 and 20 mmHg, with PMS pressures measured at pressure sensor 28 ranging from 0.1 to 200 mmHg, PLS pressures measured at pressure sensor 36 ranging from 0 to 30 psi, and DPS flow rates measured at flow meter 26 ranging from 0.1 to 50 slpm.

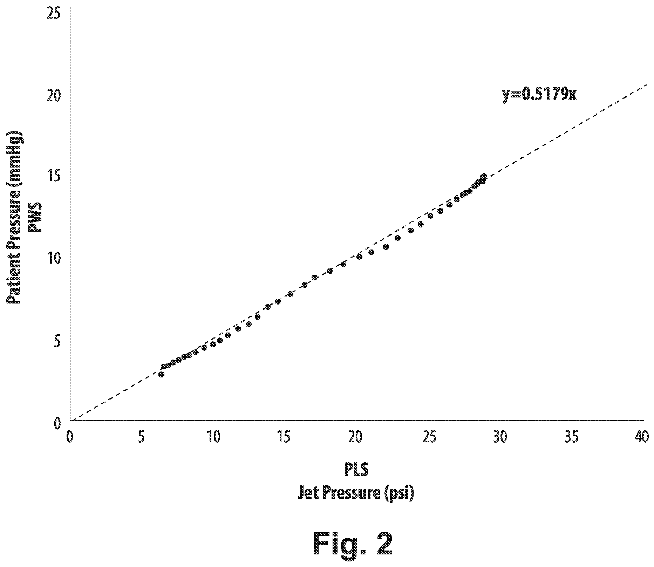

Utility of the polynomial is predicated on the consistency of the contribution of the jest in trocar 16 to cavity pressure PWS measured at sensor 38 versus the supply pressure, PLS measured at sensor 36, as shown in Fig. 2. Jet geometry, i.e. feature size and form, must be substantially equivalent across the range of access ports or trocars used within the system, or non-equivalent jet geometries must be engineered or calibrated to nominally equivalent performance. In other embodiments of the subject disclosure, the system is adapted and configured to identify a specific jet geometry within the trocar or access port via electronic sensors and applies an appropriate polynomial from a finite set of polynomials each having coefficients that are empirically derived.

Compared to prior art systems and methods, the use of a polynomial function requires less processor and memory resources. This can reduce hardware costs and/or provide for pressure calculation on shorter, more frequent intervals. Also, the elimination of lookup tables, as used in commonly assigned U.S. Patent No. 11,033,299, reduces error associated with finding the nearest table value or interpolation error. Additionally, by using experimentally derived coefficients in controlled laboratory environments followed by rigorous design verification testing, the hazards associated with establishing intra-operative tables or curves as in U.S. Patent Application 2021/0346592 to Zeyssig et al., are greatly reduced as outlier data points due to noise would pose a hazard until the dataset is next intra-operatively established.

As will be appreciated by those skilled in the art, aspects of the present disclosure may be embodied as a system, method or computer program product. Accordingly, aspects of this disclosure may take the form of an entirely hardware embodiment, an entirely software embodiment (including firmware, resident software, micro-code, etc.), or an embodiment combining software and hardware aspects, all possibilities of which can be referred to herein as a "circuit," "module," or "system." A "circuit," "module," or "system" can include one or more portions of one or more separate physical hardware and/or software components that can together perform the disclosed function of the "circuit," "module," or "system", or a "circuit," "module," or "system" can be a single self-contained unit (e.g., of hardware and/or software). Furthermore, aspects of this disclosure may take the form of a computer program product embodied in one or more computer readable medium(s) having computer readable program code embodied thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber cable, RF, etc., or any suitable combination of the foregoing.

Computer program code for carrying out operations for aspects of this disclosure may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. **In** the latter scenario, the remote computer may be connected to the user's computer through any type of network, including a local area network (LAN) or a wide area network (WAN), or the connection may be made to an external computer (for example, through the Internet using an Internet Service Provider).

Aspects of this disclosure may be described above with reference to flowchart illustrations and/or block diagrams of methods, apparatus (systems) and computer program products according to embodiments of this disclosure. It will be understood that each block of any flowchart illustrations and/or block diagrams, and combinations of blocks in any flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in any flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

While the subject disclosure has been shown and described with reference to preferred embodiments, those skilled in the art will readily appreciate that changes and/or modifications may be made thereto without departing from the scope of the subject disclosure.

**Claims**

1.  A multimodal surgical gas delivery system (10) for monitoring insufflated body cavity pressure comprising:

     a) an insufflation portion (20) defining an insufflation gas flow path (22) for receiving insufflation gas from a gas supply (12) by way of a pressure regulator (14) and for delivering the insufflation gas to a patient's body cavity by way of a gas sealed surgical access device (16), the insufflation gas flow path (22) including a gas control valve (24) downstream from the pressure regulator (14), and further including an insufflation gas flow sensor (26) and an insufflation gas pressure sensor (28) downstream from the gas control valve (24); and
     b) a gaseous sealing portion (30) defining a gas recirculation circuit (32) for communicating with the surgical access device (16) to generate a gaseous seal therein for maintaining a stable body cavity pressure, the gas recirculation circuit (32) including a compressor (34) having a discharge path (32a) and a return path (32b), wherein a discharge pressure sensor (36) is operatively associated with the compressor discharge path (32a);
     c) a processor (40) operatively associated with the insufflation portion (20) and the gaseous sealing portion (30) for repeatedly calculating body cavity pressure by way of the surgical access device (16) using a polynomial function of at least three relatively weighted terms including an insufflation gas supply (12) pressure measured by the insufflation gas pressure sensor (28), an insufflation gas flow rate measured by the insufflation gas flow sensor (26) and a compressor discharge pressure measured by the discharge pressure sensor (36), wherein the processor (40) is adapted and configured to adjust gas flow through the gas control valve (24) and the compressor (34) to maintain the body cavity pressure within a desired range.

2.  A multimodal surgical gas delivery system as recited in Claim 1, wherein the insufflation gas pressure sensor (28) is located upstream from the insufflation gas flow sensor (26).

3.  A multimodal surgical gas delivery system as recited in Claim 1, wherein the insufflation gas pressure sensor (28) is located downstream from the insufflation gas flow sensor (26).

4.  A multimodal surgical gas delivery system as recited in Claim 1, wherein the insufflation portion (20) includes two gas pressure sensors (28) including a first gas pressure sensor located upstream from the gas flow sensor (26) and a second gas pressure sensor (38) located downstream from the gas flow sensor (26).

5.  A multimodal surgical gas delivery system as recited in Claim 1, wherein the processor (40) is configured to periodically calculate body cavity pressure at a polling frequency of between 1 GHz and 0.01 Hz.

6. A multimodal surgical gas delivery system as recited in Claim 1, wherein the processor (40) is configured to periodically calculate body cavity pressure at a polling frequency of 1 kHz.

7. A multimodal surgical gas delivery system as recited in Claim 1, wherein the processor (40) is adapted and configured to adjust gas flow through the gas control valve (24) and the compressor to maintain the body cavity pressure within a range of between 0.1 and 20 mmHg.

**Patentansprüche**

1. Multimodales chirurgisches Gasabgabesystem (10) zur Überwachung des Drucks in einer insufflierten Körperhöhle, umfassend:

   a) einen Insufflationsabschnitt (20), der einen Insufflationsgasstromweg (22) bildet, um Insufflationsgas von einer Gaszufuhr (12) über einen Druckregler (14) aufzunehmen und das Insufflationsgas einer Körperhöhle eines Patienten über eine gasdichte chirurgische Zugangsvorrichtung (16) zuzuführen, wobei der Insufflationsgasstromweg (22) ein Gassteuerventil (24) stromabwärts vom Druckregler (14) umfasst und ferner einen Insufflationsgasstromsensor (26) und einen Insufflationsgasdrucksensor (28) stromabwärts vom Gassteuerventil (24) umfasst; und
   b) einen Gasdichtungsabschnitt (30), der einen Gasrückführungskreislauf (32) zur Verbindung mit der chirurgischen Zugangsvorrichtung (16) bildet, um darin eine Gasdichtung zur Aufrechterhaltung eines stabilen Körperhöhlendrucks zu erzeugen, wobei der Gasrückführungskreislauf (32) einen Kompressor (34) mit einem Abgabeweg (32a) und einem Rücklaufweg (32b) umfasst, wobei ein Abgabedrucksensor (36) mit dem Kompressorabgabeweg (32a) wirkverbunden ist;
   c) einen Prozessor (40), der mit dem Insufflationsabschnitt (20) und Gasdichtungsabschnitt (30) wirkverbunden ist, um den Körperhöhlendruck über die chirurgische Zugangsvorrichtung (16) unter Verwendung einer Polynomfunktion aus zumindest drei relativ gewichteten Termen wiederholt zu berechnen, die einen durch den Insufflationsgasdrucksensor (28) gemessenen Druck der Insufflationsgaszufuhr (12), eine durch den Insufflationsgasstromsensor (26) gemessene Insufflationsgasstrommenge und einen durch den Abgabedrucksensor (36) gemessener Kompressorabgabedruck umfasst, wobei der Prozessor (40) angepasst und eingerichtet ist, um den Gasstrom durch das Gassteuerventil (24) und den Kompressor (34) einzustellen, um den Körperhöhlendruck innerhalb eines gewünschten Bereichs aufrechtzuerhalten.

2. Multimodales chirurgisches Gasabgabesystem nach Anspruch 1, wobei der Insufflationsgasdrucksensor (28) stromaufwärts vom Insufflationsgasstromsensor (26) angeordnet ist.

3. Multimodales chirurgisches Gasabgabesystem nach Anspruch 1, wobei der Insufflationsgasdrucksensor (28) stromabwärts vom Insufflationsgasstromsensor (26) angeordnet ist.

4. Multimodales chirurgisches Gasabgabesystem nach Anspruch 1, wobei der Insufflationsabschnitt (20) zwei Gasdrucksensoren (28) mit einem ersten Gasdrucksensor, der stromaufwärts vom Gasstromsensor (26) angeordnet ist, und mit einem zweiten Gasdrucksensor (38) umfasst, der stromabwärts vom Gasstromsensor (26) angeordnet ist.

5. Multimodales chirurgisches Gasabgabesystem nach Anspruch 1, wobei der Prozessor (40) eingerichtet ist, um den Körperhöhlendruck periodisch mit einer Abfragefrequenz zwischen 1 GHz und 0,01 Hz zu berechnen.

6. Multimodales chirurgisches Gasabgabesystem nach Anspruch 1, wobei der Prozessor (40) eingerichtet ist, um den Körperhöhlendruck periodisch mit einer Abfragefrequenz von 1 kHz zu berechnen.

7. Multimodales chirurgisches Gasabgabesystem nach Anspruch 1, wobei der Prozessor (40) angepasst und eingerichtet ist, um den Gasstrom durch das Gassteuerventil (24) und den Kompressor einzustellen, um den Körperhöhlendruck in einem Bereich zwischen 0,1 und 20 mmHg aufrechtzuerhalten.

**Revendications**

1. Système multimodal d'administration de gaz chirurgical (10) pour surveiller la pression de cavité corporelle insufflée, comprenant :

   a) une partie d'insufflation (20) définissant un trajet d'écoulement de gaz d'insufflation (22) pour recevoir du gaz d'insufflation d'une alimentation en gaz (12) au moyen d'un régulateur de pression (14) et pour administrer le gaz d'insufflation à la cavité corporelle d'un patient au moyen d'un dispositif d'accès chirurgical étanche aux gaz (16), le trajet d'écoulement de gaz d'insufflation (22) comprenant une soupape de commande de gaz (24) en aval du régulateur de pression (14), et comprenant en outre un capteur d'écoulement de gaz d'insufflation (26) et un capteur de pression de gaz d'insufflation (28) en aval de la soupape de commande de gaz (24) ; et
   b) une partie d'étanchéité gazeuse (30) définissant un circuit de recirculation de gaz (32) pour communiquer avec le dispositif d'accès chirurgical (16) afin de générer une étanchéité gazeuse dedans pour maintenir une pression de cavité corporelle stable, le circuit de recirculation de gaz (32) comprenant un compresseur (34) ayant un trajet de refoulement (32a) et un trajet de retour (32b), où un capteur de pression de refoulement (36) est associé de manière fonctionnelle au trajet de refoulement (32a) du compresseur ;
   c) un processeur (40) associé de manière fonctionnelle à la partie d'insufflation (20) et à la partie d'étanchéité gazeuse (30) pour calculer de manière répétée la pression de cavité corporelle au moyen du dispositif d'accès chirurgical (16) en utilisant une fonction polynomiale d'au moins trois termes relativement pondérés comprenant une pression d'alimentation en gaz d'insufflation (12) mesurée par le capteur de pression de gaz d'insufflation (28), un débit de gaz d'insufflation mesuré par le capteur d'écoulement de gaz d'insufflation (26) et une pression de refoulement de compresseur mesurée par le capteur de pression de refoulement (36), où le processeur (40) est adapté et configuré pour ajuster le flux de gaz à travers la soupape de commande de gaz (24) et le compresseur (34) pour maintenir la pression de cavité corporelle dans une plage souhaitée.

2. Système multimodal d'administration de gaz chirurgical selon la revendication 1, dans lequel le capteur de pression de gaz d'insufflation (28) est situé en amont du capteur d'écoulement de gaz d'insufflation (26).

3. Système multimodal d'administration de gaz chirurgical selon la revendication 1, dans lequel le capteur de pression de gaz d'insufflation (28) est situé en aval du capteur d'écoulement de gaz d'insufflation (26).

4. Système multimodal d'administration de gaz chirurgical selon la revendication 1, dans lequel la partie d'insufflation (20) comprend deux capteurs de pression de gaz (28) comprenant un premier capteur de pression de gaz situé en amont du capteur d'écoulement de gaz (26) et un second capteur de pression de gaz (38) situé en aval du capteur d'écoulement de gaz (26).

5. Système multimodal d'administration de gaz chirurgical selon la revendication 1, dans lequel le processeur (40) est configuré pour calculer périodiquement la pression de cavité corporelle à une fréquence d'interrogation comprise entre 1 GHz et 0,01 Hz.

6. Système multimodal d'administration de gaz chirurgical selon la revendication 1, dans lequel le processeur (40) est configuré pour calculer périodiquement la pression de cavité corporelle à une fréquence d'interrogation de 1 kHz.

7. Système multimodal d'administration de gaz chirurgical selon la revendication 1, dans lequel le processeur (40) est adapté et configuré pour ajuster le flux de gaz à travers la soupape de commande de gaz (24) et le compresseur pour maintenir la pression de cavité corporelle dans une plage comprise entre 0,1 et 20 mmHg.

**Fig. 1**

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63395597
- US 10639434 B
- US 11033299 B, Silver
- US 20210346592 A, Zeyssig
- US 2018221598 A1
- US 9199047 B
- US 8795223 B
- US 9907569 B